Europäisches Patentamt

European Patent Office

Office européen des brevets

(11) Numéro de publication: **0 214 017 B1**

# FASCICULE DE BREVET EUROPEEN

(45) Date de publication de fascicule du brevet: **22.07.92** (51) Int. Cl.5: **A61K 31/155**

(21) Numéro de dépôt: **86401717.3**

(22) Date de dépôt: **31.07.86**

(54) **Utilisation du p-chlorophénoxyacétate de N,N-diméthylbiguanide pour le traitement des neuropathies et de la dégénérescence nerveuse.**

(30) Priorité: **31.07.85 FR 8511664**

(43) Date de publication de la demande:
**11.03.87 Bulletin 87/11**

(45) Mention de la délivrance du brevet:
**22.07.92 Bulletin 92/30**

(84) Etats contractants désignés:
**AT BE CH DE FR GB IT LI LU NL SE**

(56) Documents cités:
**DE-A- 1 900 772**

**ACTA MED SCAND, vol. 213, 1983, pages 283-287; C.-D. AGARDH et al.: "Improvement of peripheral nerve function after institution of insulin treatment in diabetes mellitus"**

**J. PHARM. CLIN., vol. 3, no. 3-4, 1984, pages 243-282; J. HUET: "Les antidiabétiques actifs par voie orale "**

(73) Titulaire: **LABORATOIRES ANPHAR-ROLLAND
Tour Lorraine France-Evry
F-91000 Evry Ville Nouvelle(FR)**

(72) Inventeur: **Thal, Claude
17 Rue du Clos Saint Marcel
F-92330 Sceaux(FR)**
Inventeur: **Hugelin,Andre
34 rue Pierret
92200 Neuiilly s/Seine(FR)**

(74) Mandataire: **Burtin, Jean-François
Cabinet GEFIB, 59, rue Edouard-Vaillant
F-92300 Levallois-Perret(FR)**

**Description**

La présente invention a pour objet l'utilisation d'un composé pharmaceutique pour la réalisation d'un médicament à visée neurotrophique.

Plus particulièrement, l'invention se rapporte à l'utilisation d'un composé pharmaceutique pour la réalisation d'un médicament destiné à permettre le développement, la maintenance et la régénération des cellules nerveuses, et une protection contre les neuropathies et les dégénérescences nerveuses.

Spécifiquement l'invention a pour objet l'utilisation du p-chlorophenoxy-acétate de de NN-diméthyl-biguanide pour la réalisation d'un médicament apte à prévenir ou à traiter les neuropathies après lésion d'origine thérapeutique, immunologique, virale ou traumatique chez des sujets diabétiques ou non-diabétiques. Le médicament renferme une quantité neurologiquement active de p-chlorophénoxy-acétate de N,N-diméthyl-biguanide en association ou en mélange avec un excipient ou un véhicule inerte, non toxique, pharmaceutiquement acceptable.

On connaissait déjà des compositions pharmaceutiques à base de p-chlorophénoxy-acétate de N,N-diméthyl-biguanide. Elles étaient utilisées sous une forme et à une posologie appropriée pour le traitement de certaines formes du diabète et, notamment, du diabète non insulino-dépendant. Dans ces formes la quantité de principe actif est de 500 mg et la posologie habituelle est de 1 000 à 1 500 mg par jour, par voie buccale.

Or, on sait qu'une des complications du diabète réside dans l'apparition de dégénérescences nerveuses que les médicaments classiques du diabète ne parviennent pas à contrôler ou à traiter.

On vient de trouver, et c'est là que se situe l'invention, que le p-chlorophénoxy-acétate de N,N-diméthyl-biguanide possède des propriétés neurotrophiques qui contribuent à combattre ou à diminuer les complications nerveuses graves du diabète sans toutefois interférer avec le traitement propre de cette maladie.

Compte tenu des résultats obtenus au cours de l'expérimentation pharmacologique, on est en droit de penser, en outre, que chez le sujet non-diabétique le p-chlorophénoxy-acétate de N,N-diméthyl-biguanide manifeste également cet effet neurotrophique qui autorise son utilisation dans le traitement de toute une gamme de neuropathies d'origine nerveuse ou traumatique.

L'invention consiste donc dans le fait que l'utilisation du p-chlorophénoxy-acétate de N,N-diméthyl-biguanide pour la zéalisation d'un médicament trouve un emploi dans le traitement des neuropathies d'origine diabétique ou non diabétique, traumatique, toxique ou dégénérative et assure la régénération des fibres nerveuses après lésion de toute origine thérapeutique, immunologique, virale.

On peut également prévoir que l'utilisation selon l'invention pourrait servir pour la prévention ou le traitement des neuropathies entraînant une lésion des axones, par certains toxiques tels que les alcaloïdes de la pervenche (Vinca Rosea), l'Almitrine, la colchicine, et la plupart des médicaments neurotoxiques.

Cet effet nouveau est obtenu par une posologie nettement différente de celle utilisée précédemment. Il est nécessaire de recourir à des posologies sensiblement plus élevées, s'échelonnant chez l'adulte entre 1,50 et 4 g de principe actif par jour. En outre, l'administration n'est pas exclusivement digestive. Elle est aussi parentérale ou rectale.

Les compositions pharmaceutiques contiennent donc une quantité neurologiquement active de p-chlorophénoxy-acétate de N,N-diméthyl-biguanide comprise entre 1 000 et 2 500 mg par prise unitaire.

Les formes pharmaceutiques les plus appropriées sont les comprimés effervescents, les tablettes, les comprimés à mâcher, les ampoules buvables ou les solutés buvables.

Parmi les véhicules ou les excipients, on pourra citer plus particuliérement ceux qui conviennent pour l'administration par voie parentérale ou par voie digestive; On pourra citer à cet égard l'eau, les solutés de chlorure de sodium, les solutions isotoniques, les solutés de polyéthylène glycol dans l'eau, les solutions aqueuses de polyvinylpyrrolidone pour les formes injectables, les diluants usuels comme les amidons, les celluloses, le carbonate de magnésium, le phosphate de calcium, la silice, le stéarate de magnésium, le talc; les agents liants, les agents édulcorants, parfumants ou de sapidité pour les formes solides. On peut également utiliser des formes appropriées pour l'administration par voie rectale comme les suppositoires ou les capsules rectales.

Le procédé d'obtention des compositions pharmaceutiques à visée neurotrophique est caractérisé en ce qu'on mélange ou ajoute une quantité neurologiquement active de p-chlorophénoxy-acétate de N,N-diméthyl-biguanide à un excipient ou à un véhicule inerte non-toxique pharmaceutiquement acceptable.

Cette incorporation ou ce mélange s'effectue selon les méthodes usuelles de la pharmacotechnie.

La partie expérimentale qui figure ci-après fournit un résumé des essais pratiqués sur l'animal et illustre l'invention.

EXEMPLE I

Comprimés effervescents de p-chlorophénoxy-acétate de N,N-diméthyl-biguanide:

| p-chlorophénoxy-acétate de N,N-diméthyl-biguanide | 1 500 g |
|---|---|
| amidon de maïs | 24 g |
| amidon de blé | 36 g |
| lactose | 375 g |
| bicarbonate de sodium | 12,6 g |
| acide tartrique | 11,25 g |
| hydroxypropylcellulose | 18 g |
| polyvinylpyrrolidone commercialisée sous la marque POVIDONE C 15 | 12 g |
| sucre glace | 120 g |
| pour 1 000 comprimés terminés à | 2,1 g |

EXEMPLE II

L'expérimentation a été conduite sur quatre lots de dix rats Whistar mâles de 220 g ± 5. Les animaux ont été opérés au jour J0, sous anesthésie au pentobarbital (40 mg/kg I.P.). Le nerf sciatique était congelé en trois points équidistants sur la même circonférence d'un nerf, au tiers moyen de la cuisse, au moyen d'une cryo-sonde portée à -170°C pendant trois secondes. Les traitements ont été commencés au jour J +1. Les animaux ont été réopérés au jour J +7 sous anesthésie au Nembutal (20 mg/kg I.P.), en supprimant ni le tonus des muscles de la queue, ni les réflexes nociceptifs de défense.

La régénération des fibres sensitives était mesurée au jour J +7, après dissection du nerf sciatique sur tout son trajet, de la cheville à la lésion. La régénération des fibres sensitives était testée en stimulant mécaniquement le nerf, au moyen d'une pince mousse en remontant le long du nerf dans le sens distoproximal. La longueur des fibres régénérées était évaluée en mesurant la position du point à partir duquel la première réponse réflexe nociceptive était obtenue.

Les mesures ont été réalisées sur des groupes d'animaux recevant par voie I.P. et à volume égal (1 ml):
1. du sérum physiologique
2. une solution non tamponnée contenant 145 mg/kg d'Isaxonine
3. une solution contenant 250 mg/kg de p-chlorophénoxy-acétate de N,N-diméthyl-biguanide
4. une solution contenant 250 mg/kg de chlorhydrate de N,N-diméthyl-biguanide

Les longueurs régénérées ont été les suivantes:

| témoins | 22,15 mm ± 0,98 S.D. |
|---|---|
| Isaxonine | 23,75 mm ± 1,03 S.D. |
| phénoxyacétate de diméthyl-biguanide | 23,87 mm ± 1,45 S.D. |
| chlorhydrate de N,N-diméthyl-biguanide | 23,00 mm ± 1,26 |

La comparaison des moyennes sur un test "T" indique que la régénération est significativement augmentée (+ 7%) avec le phénoxyacétate. Les résultats ne sont pas significatifs pour le chlorhydrate de N,N-diméthyl-biguanide. Il n'existe pas de différence significative entre la régénération des fibres sensitives des rats traités par l'Isaxonine et le N,N-diméthyl-biguanide.

Ceci indique une augmentation de la longueur régénérée de 7%. La régénération est significativement augmentée par détermination du test "T" qui ne s'applique pas à la valeur de 7%.

EXEMPLE III

Etude de la vitesse de réinnervation sensitive chez le rat.

La vitesse de réinnervation sensitive après lésion du nerf sciatique et dégénérescence wallérienne a été étudiée chez le rat Whistar mâle de 210 g ± 10 g. Le nerf sciatique droit a été lésé par congélation au moyen d'une cryode à cataracte portée à -170°C pendant 30 secondes en 3 points d'une même circonférence située au tiers supérieur de la cuisse, à 90 ± 5 mm de la pénétration du nerf dans la région

3

plantaire. Le nerf femoris interne était excisé sur plusieurs centimètres. La récupération sensitive a été appréciée en mesurant la latence du potentiel évoqué somesthésique recueilli au niveau de l'aize primaire contralatérale et homolatérale, au moyen d'électrodes métalliques extraduremériennes scellées et laissées à demeure. La réponse était évoquée en stimulant le coussinet le plus interne de la sole plantaire droite au moyen d'un choc électrique de 5 mA délivré à courant constant pendant 2 ms toutes les 620 ms. Les réponses étaient moyennées de 500 à 2 000 fois et leurs latences mesurées avec une précision de ± 0,5 ms.

La latence de la réponse positive (P1) la plus précoce et à seuil le plus bas a été mesurée et comparée chez deux groupes d'animaux aux jours J + 25, J + 29, J + 33, J + 38 et J + 43. Le groupe de témoins (N = 11) a reçu par gavage 6 jours sur 7 0,5 g/kg de gomme arabique. Le groupe traité a reçu, aux mêmes dates et par la même voie, l'un (M = 6) 0,5 g/kg de parachlorophenoxy-acétate de diméthyl-biguanide. Les courbes de régression ont été calculées par la méthode des moindres carrés et les moyennes ont été comparées par couple à l'aide du test "t" de student.

Les résultats obtenus indiquent une accélération de la vitesse de réapparition des réponses sensitives P1 de 2,5 jours en moyenne chez les animaux traités par parachlorophénoxy-acétate de diméthyl-biguanide.

Les moyennes sont significativement différentes pour chaque point de la courbe. Entre le lot des animaux traités et celui des animaux témoins, la comparaison des moyennes par couple donne une différence significative au niveau P < 0,01 entre les témoins d'une part et le groupe traité d'autre part.

Le traitement par le parachlorophénoxy-acétate de N,N-diméthyl-biguanide fait apparaître précocement, dès le jour J + 25, une réponse négative N1 à seuil plus élevé que P1 qui ne peut être observée chez les animaux témoins avant le 50ème jour après la lésion.

Les résultats obtenus conduisent à la conclusion que le parachlorophénoxy-acétate de N,N-diméthyl-biguanide a une action puissante sur la régénération des fibres nerveuses du mammifère.

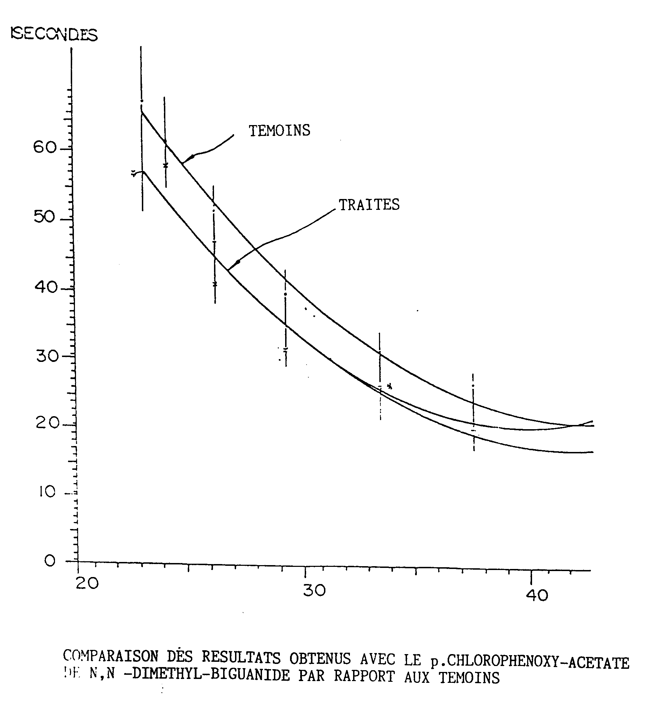

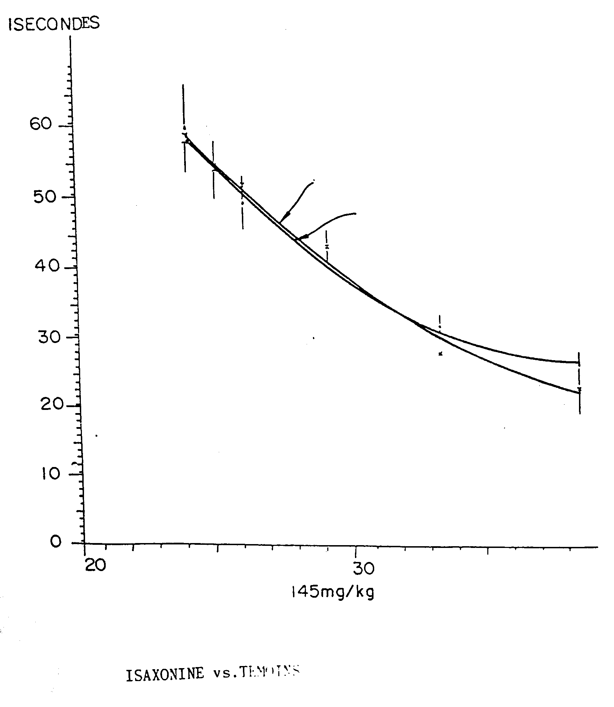

## Revendications

1. Utilisation du p-chlorophenoxy-acétate de NN-diméthyl-biguanide pour la réalisation d'un médicament apte à prévenir ou à traiter les neuropathies après lésion d'origine thérapeutique, immunologique, virale ou traumatique chez des sujets diabétiques ou non-diabétiques.

2. Utilisation selon la revendication 1, dans laquelle la quantité de principe actif s'échelonne de 1000 à 2500 mg par prise unitaire.

## Claims

1. Utilization of NN-dimethyl-biguanide p-chlorophenoxy-acetate in view of the realization of a medicine suitable for preventing or treating neuropathias after damage of therapeutic, immunologic, viral or traumatic origin in diabetic or non-diabetic patients.

2. Utilization according to claim 1. wherein the amount of active ingredient ranges from 1000 to 2500 mg per unit dosage.

## Patentansprüche

1. Verwendung NN-dimethyl-biguanid p-chlorophenoxy-azetats zur Herstellung eines Arzneimittels fur das Vorbeugen oder das Behandeln Neuropathias nach Störungen von therapeutischen, immunologischen, viralen oder traumatischen Ursprung bei diabetischen oder non-diabetischen Subjekten, geeignet.

2. Verwendung nach Anspruch 1, worin die Menge an Wirkstoff von 1000 bis 2500 mg per Einzelndosis sich verteilt.

COMPARAISON DES RESULTATS OBTENUS AVEC LE p.CHLOROPHENOXY-ACETATE DE N,N -DIMETHYL-BIGUANIDE PAR RAPPORT AUX TEMOINS

ISAXONINE vs. TEMOINS